(19) Europäisches Patentamt
European Patent Office
Office européen des brevets

(11) **EP 4 613 835 A1**

(12) **EUROPEAN PATENT APPLICATION**

(43) Date of publication:
**10.09.2025 Bulletin 2025/37**

(21) Application number: **24161084.9**

(22) Date of filing: **04.03.2024**

(51) International Patent Classification (IPC):
*C11D 3/20* (2006.01)   *A61K 8/365* (2006.01)
*A61P 17/10* (2006.01)   *A61Q 5/00* (2006.01)
*A61Q 5/12* (2006.01)   *A61Q 19/10* (2006.01)
*C11D 7/26* (2006.01)   *D06M 13/207* (2006.01)

(52) Cooperative Patent Classification (CPC):
**C11D 3/2086; A61K 8/365; A61P 17/10;
A61Q 5/00; A61Q 5/02; A61Q 5/12; A61Q 19/10;
C07C 62/32; C11D 7/265; D06M 13/207;
C07C 2601/16; C11D 2111/12; C11D 2111/14**

(84) Designated Contracting States:
**AL AT BE BG CH CY CZ DE DK EE ES FI FR GB
GR HR HU IE IS IT LI LT LU LV MC ME MK MT NL
NO PL PT RO RS SE SI SK SM TR**
Designated Extension States:
**BA**
Designated Validation States:
**GE KH MA MD TN**

(71) Applicant: **SkyLab AG**
**1066 Epalinges (CH)**

(72) Inventors:
• Belous, Elena Yur'evna
121309 MOSCOW (RU)
• Filatov, Viktor Andreevich
119415 MOSCOW (RU)

(74) Representative: **Glawe, Delfs, Moll
Partnerschaft mbB
Hopfenmarkt 33
20457 Hamburg (DE)**

(54) **NOVEL USE OF SHIKIMIC ACID IN HOUSEHOLD CHEMICALS PROVIDING LONGLASTING CLEANLINESS AND HEALTHFUL CLEANSING**

(57)    The invention relates to the use of shikimic acid in the manufacture of a cleaning composition and a cleaning composition comprising 0.001-0.50% wt. of said shikimic acid and wherein the pH of said cleaning composition is in the range of 2.5-12.

Laundry gel viscosity versus shikimic acid concentration after storage at +40°C for 25 days.
SA – shikimic acid.

**Fig. 1**

EP 4 613 835 A1

**Description**

FIELD OF THE INVENTION

The invention is related to shikimic acid and the use thereof for manufacture of the detergents for cleaning dense or hard surfaces, including but not limited to liquid, powder, capsule laundry washing detergents, laundry conditioners for fabric softening and freshening, and the surface washers and cleaners. The use of the composition allows to manufacture highly efficient household chemicals, to stabilize said chemicals with no need for additional active ingredients, and to provide hygienic effect to maintain the health of consumers. The invention can also be implemented as a shampoo, a hair conditioner or a balm, and also as acne or pimple cleaner comprised by a patch. The use of shikimic acid contributes to lower bacterial and fungal burden in the consumer, regulates aromatic environment indoors, keeps long-lasting cleanliness and is safe for the skin.

BACKGROUND OF THE INVENTION

In view of everyday interaction of clothes with body and environment, the development of laundry washing detergents and conditioners having improved properties is of importance *[The interview of 1933 persons through Kantar Profiles|Mintel; A year of innovation in fabric and dish care, 2021, Mintel]*. The search for effective active ingredients capable of successful control of contaminants is a promising and relevant field. Natural agents, which are both efficient surface cleaners and environmental and human friendly are of special interest.

Shikimic acid is one of such agents. Shikimic acid is a low-molecular weight compound, and can be produced by chemical synthesis, microbiological fermentation, and extraction from certain plant species, in particular, from the seeds of star-anise (Latin *Illicium verum*). Despite the presence in many microorganisms and fungi shikimic acid is an intermediate metabolite of biosynthetic reactions and is found in microorganisms and fungi at very low levels. Significant amount of shikimic acid can be found only in star-anise and related plants [Ghosh S. et al., Production of shikimic acid //Biotechnology advances, 2012*]*.

Shikimic acid is best known in beauty products, for instance, in hair and scalp products, but is not used in the household chemicals. "Purifying Shampoo with Water and Vitamin E" by L'Erbolario comprising shikimic acid as an ingredient is known. The claimed effects of the shampoo include deep cleansing effect and washing away unpleasant odors [https://www.erbolario.com/en/shampoo-/purifying-shampoo-with-water-mint-and-vitamin-e-2937.html]. The manufacturer does not specify the agent removing unpleasant odors, while as follows from information on the manufacturer's website, the main active ingredients include rosemary extract, vitamin E of soybean origin, water mint, and pepper mint. Shikimic acid is used as alpha-hydroxy acid (AHA) for skin peeling, as acne-preventing ingredient and as skin-bleaching agent [Batory M, Rotsztejn H. Shikimic acid in the light of current knowledge. J Cosmet Dermatol. 2022 Feb;21(2):501-505].

However, direct deodorizing effect of shikimic acid in respect to unpleasant odors has not been disclosed. Shikimic acid prevents formation of unpleasant body odors by lipase inhibition rather than eliminates the odors. Lipase is a bacterial enzyme that splits compound fats to fatty acids and unpleasantly smelling aldehydes. Thus, shikimic acid prevents unpleasant odor formation through inhibition of bacterial enzyme, which correlates with the odor emergence [Munk S. et al. Primary odorants of laundry soiled with sweat/sebum: Influence of lipase on the odor profile, 2000*; Batory M., Rotsztejn H. Shikimic acid in the light of current knowledge, 2022]*.

Detox peeling for scalp "Ice by Natura Siberica refresh my scalp peeling" by Natura Siberica is another hair care product comprising shikimic acid. The manufacturer claims that shikimic acid exhibits adsorbing and exfoliative effects without causing skin irritation [*https://goldapple.ru/19000002943-ice-professional-by-ns-refresh-my-scalp-peeling*].

Shikimic acid is also used in the other beauty products, in particular, in deodorants. "Shikimic acid deodorant roll-on" by Earth Rhythm is one of such products. According to information by manufacturer, shikimic acid provides control and prevention of unpleasant odor generation, acts as powerful but not irritating exfoliating agent for sensitive skin; naturally minimizing unpleasant body odor due to antimicrobial effect [*https://earthrhythm.com/products/shikimic-acid-deodorant*].

Antibacterial properties of shikimic acid were investigated in respect to several bacterial strains including *Staphylococcus aureus* and *Cutibacteium acnes*. Shikimic acid causes damage of cytoplasmic membrane, affects ion balance by interaction with potassium and calcium channels. Upon intercellular penetration shikimic acid disturbs the protein synthesis and normal bacterial metabolism [Bai J. et al. Antibacterial activity of shikimic acid from pine needles of Cedrus deodara against Staphylococcus aureus through damage to cell membrane, 2015*; Bai J. et al. Comparative study on antibacterial mechanism of shikimic acid and quinic acid against Staphylococcus aureus through transcriptomic and metabolomic approaches, 2022]*.

Summing up, shikimic acid is widely used as an ingredient of beauty products acting as non-aggressive exfoliating agent and antibacterial. Further, shikimic acid is widely used as a substrate for chemical synthesis of the drug compounds, in particular, of oseltamivir, commercial name Tamiflu®. Oseltamivir is an effective inhibitor of several influenza strains and is extensively used as the first choice therapy for influenza caused by various strains of that RNA virus [Borah J. C. Shikimic acid: a highly prospective molecule in pharmaceutical industry, 2015].

Shikimic acid is rarely used as a monocomponent - usually it is used in combination with the other compounds potentiating its activity. The efficacy of shikimic acid in beauty products is greater when used in combination with other acids, in particular, with lactic acid, glycolic acid, lactobionic acid, ferulic acid, succinic acid, malic acid, azelaic acid, and gluconolactone [Batory et al., 2022].

Nevertheless, the use of shikimic acid as an ingredient of household chemicals intended for preparation of laundry washing products, such as laundry detergents or conditioners has not been previously disclosed. Shikimic acid instability in formulations is the most probable explanation of the fact that it has not been used as the ingredient of household chemicals. Many components of natural origin and having pharmaceutical potential show low stability because of oxidation or intermolecular reactions [Taofiq O. et al. Hydroxycinnamic acids and their derivatives: Cosmeceutical significance, challenges and future perspectives, a review, 2017].

Low stability of polyphenols and hydroxycinnamic acids, which are the related compounds to shikimic acid, is reported in literature. Encapsulation of those compounds and also of phenolic acids as such, including shikimic acid, is one of the methods leading to stabilization [Bartosz T., Irene T. Polyphenols encapsulation application of innovation technologies to improve stability of natural products, 2016; Taofiq O. et al., 2017; Taofiq O. et al. Phenolic acids, cinnamic acid, and ergosterol as cosmeceutical ingredients: Stabilization by microencapsulation to ensure sustained bioactivity, 2019]. Furthermore, it is known that alpha-hydroxy acids (AHA) exhibit negative effect on activity of enzymes, which are the principal components of the household chemicals and stain removers. In particular, shikimic acid inhibits amylase, which is used for removing starch-based spots [Cao J. et al. Elucidation of alpha-amylase inhibition by natural shikimic acid derivates regarding the infrequent uncompetitive inhibition mode and structure-activity relationship, 2023], lipase splitting high-fat spots [Rawat G. et al. An interactive study of influential parameters for shikimic acid production using statistical approach, scale up and its inhibitory action on different lipases, 2013]. The listed enzymes along with protease and cellulase are the principal part of household chemicals and stain removers. Thanks to acidic properties (pKa = 4.15), shikimic acid is able to decrease pH of the product leading to destabilization thereof [Batory M., Rotsztejn H. Shikimic acid in the light of current knowledge, 2022]. Thus, the use of shikimic acid as an ingredient of the household chemicals is limited by its stability and its influence on basic components of formulations of the household chemicals, which potentially impair the cleansing efficacy and stain removing efficacy.

Despite the absence of information in literature and potential conflict between shikimic acid and the components of the household chemicals, the Authors of invention unexpectedly found synergetic effect of the use of shikimic acid in the composition of various household chemicals as a stabilizer and viscosity regulator. The technical result of the use of shikimic acid is effective cleansing of various dense and hard surfaces including fabrics, in neutralization of aromatic compounds belonging to various classes of organic substances: aldehydes, ketones, terpenes, amines, indole, serum compounds (mercaptans), organic acids and organic acid esters, phenols, and cresols. Shikimic acid is effective in the pH range from 2.5 to 12 in the presence of various synthetic and natural components, thus enabling to use said agent in the compositions of the wide range of household chemicals for laundry washing of various fabrics and for cleaning of various surfaces. This agent is targeted on complex biological odors and soils on various surfaces and also binds releasing metabolites having human-offensive odors. The distinguishing feature is that shikimic acid at claimed concentrations, pH range, and as ingredient of various household chemicals improves consumer properties without compromising the appearance of the majority dense surfaces, including textile (cotton, synthetic, linen, mixed fabrics, delicate fabrics such as wool, silk, cashmere), wooden, metallic, plastic, marble, porcelain, ceramic, glass surfaces.

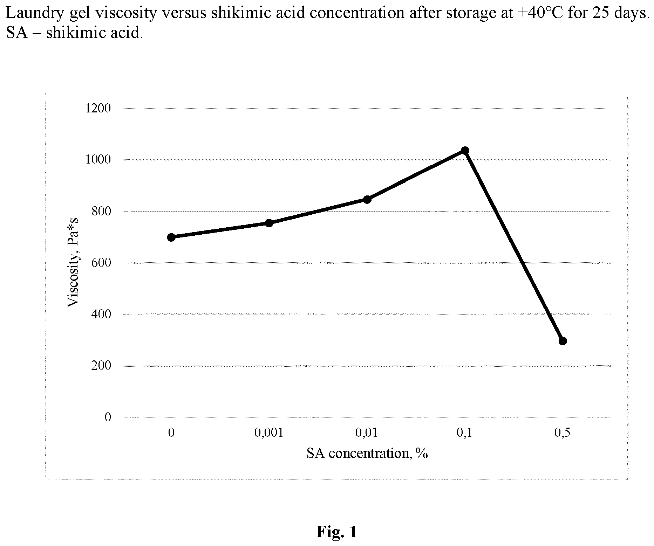

In relation to gel viscosity authors of the invention managed to discover unexpected technical result - shikimic acid in the concentration range of 0,001% to 0,5% inclusively and and in pH range from 2.5 to 12 inclusively promoted gel thickening and acted as a formulation stabilizer. Change of gel viscosity depending on shikimic acid concentration is shown in Fig.1 for accelerated aging samples. It is evident that gel viscosity increases with the addition of shikimic acid up to 0,1%. At the concentration of 0,5% viscosity drops but still stays in the viscosity normal ranges described above.

Thus, the invention is generally related to shikimic acid and the use thereof as an ingredient of various household chemicals to achieve the technical results such as stabilization of the household chemical formulations under stress stability, effective removal of various biostains, odor neutralization on various fabrics not associated with bacterial activity, with provision of long-lasting cleanliness, which is not achieved or not adequately achieved with the use of modern commercially available products in this field.

SUMMARY OF THE INVENTION

In the first aspect the invention is related to shikimic acid for use in the composition of household chemicals intended for laundry washing and for cleaning the outer dense or hard surfaces, wherein said cleansing chemical is active at pH ranging from 2.5 to 12.

In the second aspect the invention is related to the use of shikimic acid of the invention for manufacture of the washing detergent of the invention.

In the third aspect the invention is related to the surface cleaner of dense or hard surfaces, said surface cleaner comprising shikimic acid of the invention.

The invention can be distinguished by that said shikimic acid is presented as aqueous glycerol, aqueous sorbitol, or aqueous propylene-glycol or other solutions, and also as a powder, granulate formulations with various fillers. Dry shikimic acid is presented by commercially available products or standardized plant extracts.

The composition can be distinguished by any additives, which are the basic ingredients of the household chemicals: anionic surface-active agents (often abbreviated to surfactants), amphoteric surfactants, cationic surfactants, solvents, solubilizers, emulsifiers, stabilizers, thickeners, bleaching agents, preservatives, pH adjusting agents, perfume agents, essential oils.

In the other aspect the invention is related to a laundry washing detergent, comprising 0.001-0.50% wt. of composition of the invention.

In another aspect the invention is related to the detergent for various surfaces comprising 0.001-0.50% wt. of composition of the invention.

A detergent can be presented by a laundry washing detergent.

A laundry washing detergent can be distinguished by that said laundry washing detergent is selected from a detergent for delicate fabrics and a detergent for children clothes.

A laundry washing detergent can be distinguished by that said laundry washing detergent is selected from a detergent for fabric membrane and a detergent for sport clothes.

A laundry washing detergent can be distinguished by that said laundry washing detergent is a detergent powder.

A laundry washing detergent can be distinguished by that said laundry washing detergent is a liquid, dry or concentrated stain remover or bleaching agent.

A laundry washing detergent can be distinguished by that said laundry washing detergent is a laundry conditioner and refresher.

A washing detergent may be presented by liquid, foam or concentrated dishwashing formulation.

A washing detergent may be presented by soap formulation.

A washing detergent may be presented by the formulation for cleaning bathroom, kitchen, floor, toilet pan, washbowl, glass or children's rooms.

In the other aspect the invention is related to the use of shikimic acid of the invention for stabilization of the household chemical formulations under stress stability, for effective removal of various biostains, for odor neutralization on various fabrics not associated with bacterial activity, with provision of long-lasting cleanliness, hygienic cleansing of the surfaces.

The present invention may be embodied in, besides a laundry washing detergent, a surface cleaner, a dishwashing cleaner, or laundry conditioner, also in the form of, but not limited to, a hair and body cleaner, including for a living or non-living subject's hair and body cleaning and an acne or pimple cleaner, including for a mammal, wherein said mammal is preferably a human.

The present invention will be further disclosed in details by the particular embodiments and illustrated by the examples.

DETAILED DESCRIPTION OF THE INVENTION

In the first aspect the invention is related to shikimic acid for use in the composition of household chemicals intended for laundry washing and for cleaning the outer dense or hard surfaces, wherein said cleansing chemical is active at pH ranging from 2.5 to 12.

In the second aspect the invention is related to the use of shikimic acid of the invention for manufacture of the washing detergent of the invention.

The compositions of the invention can comprise shikimic acid in the range from 0.001 to 0.5% wt. depending on type of the product and intensity of desired properties. For instance, the composition can comprise shikimic acid in the amount of 0.001% wt., 0.005% wt., 0.01% wt., 0.025% wt., 0.05% wt., 0,1% wt., 0,25% wt., 0,3% wt., 0,4% wt. or 0.5 % wt. or any values in between.

The invention can be distinguished by that said shikimic acid is presented as aqueous glycerol, aqueous sorbitol, or aqueous propylene-glycol or other solutions, and also as a powder, granulate formulations with various fillers. Dry shikimic acid can be presented by the substance or commercially available product with CAS No 138-59-0.

The present invention is also related to the use of composition of the invention in the household chemical.

A household chemical of the invention can comprise a composition of the invention in the range from 0.001 to 0.5% wt. For instance, a household chemical of the invention can comprise the composition of the invention in the amount of 0.001% wt., 0.0025% wt., 0.005% wt., 0.01% wt., 0.015% wt., 0.02% wt., 0.025% wt., 0.03% wt., 0.035% wt., 0.04% wt., 0.045% wt., 0.05% wt., 0.1% wt., 0.15% wt., 0.2% wt., 0.25% wt., 0.3% wt., 0.4% wt. or 0.5% wt. or any values in between.

The present invention is also related to the laundry washing detergent, laundry washing detergent for sport clothes or laundry washing detergent for children's clothes, comprising a composition of the invention in the range from 0.001 to 0.5% wt.

The present invention is also related to powder washing detergent for white and color clothes comprising a composition of the invention in the range from 0.001 to 0.5% wt.

The present invention is also related to liquid, foam or concentrated dishwashing formulation comprising a composition of the invention in the range from 0.001 to 0.5% wt.

The present invention is also related to stain remover or bleaching agent comprising a composition of the invention in the range from 0.001 to 0.5% wt.

The present invention is also related to the soap formulation comprising a composition of the invention in the range from 0.001 to 0.5% wt.

The present invention is also related to the formulation for cleaning bathroom, kitchen, floor, toilet pan, washbowl, glass or children's rooms, comprising a composition of the invention in the range from 0.001 to 0.5% wt.

The present invention is also related to the use of shikimic acid of the invention for stabilization of the household chemical formulations under stress stability, for effective removal of various biostains, for odor neutralization on various fabrics not associated with bacterial activity, with provision of long-lasting cleanliness, hygienic cleansing of the surfaces.

The composition preferably does not comprise any other active ingredients and/or additives, such as active washing agents and/or acceptable additives; however, the composition can comprise such agents. Such agents can be presented or are presented by the agents conventionally used in this field and well known to those skilled in the art. Addition of the above agents to formulation of the invention does not cancel the achievement of the claimed technical results but rather can improve thereof.

The scope of invention also covers such household chemicals as laundry washing detergents, laundry washing detergent for sport clothes, laundry washing detergent for children's clothes, powder washing detergent.

In the present disclosure all weight ratios, weight parts, weight percent as well as volume ratios, volume parts, and volume percent are given in relation to the formulation, agent, composition, or product according to the context.

In the household chemical of the invention the acceptable additives are selected from the following categories of components.

Anionic surfactants:

Alkyl polyethylene glycol sulphate of general formula $R^1$-O(-$CH_2$-$CH_2$-O)$n^1$ ($SO_3$) $n^2$ $X^1$, wherein $n^1$ is in the range from 0 to 10 and indicates the number of polyethylene groups, $R^1$ is alkyl and/or alkenyl with hydrocarbon chain length from 5 to 22 carbon atoms, $n^2$ is in the range from 0 to 1 and indicates the number of sulphate groups, $X^1$ is a cation of alkali metal and/or alkaline-earth metal, ammonium, alkyl ammonium, alkanol ammonium, glucoammonium, basic amino acid;

Salts of higher carboxylic acids of general formula: $R^1$-$CO_2X^1$, wherein $R^1$ is alkyl and/or alkenyl with hydrocarbon chain length from 5 to 21 carbon atoms, and $X^1$ is a cation of alkali metal and/or alkaline-earth metal, ammonium, alkyl ammonium, alkanol ammonium, glucoammonium, basic amino acid;

Alkyl polyethylene glycol carboxylate of general formula: $R^5$-O(-$CH_2$-$CH_2$-O-)$n^2CH_2$-$CO_2X^5$, wherein $n^2$ is in the range from 1 to 15, and indicates the number of polyethylene glycol groups, $R^5$ is alkyl and/or alkenyl with hydrocarbon chain length from 6 to 22 carbon atoms, and $X^5$ is a cation of alkali metal and/or alkaline-earth metal, ammonium, alkyl ammonium, alkanol ammonium, glucoammonium;

Alkyl sulphate of general formula $R^3$-$OSO_3X^3$, wherein $R^3$ is alkyl and/or alkenyl with hydrocarbon chain length from 6 to 22 carbon atoms, and $X^3$ is a cation of alkali and/or alkaline-earth metal, ammonium, alkyl ammonium, alkanol ammonium, glucoammonium;

Amide salt of higher fatty acid and methylglycine of general formula $R^4$-C(O)-N(-$CH_3$)-$CH_2$-$CO_2X^4$, wherein $R^4$ is alkyl and/or alkenyl with hydrocarbon chain length from 5 to 21 carbon atoms, and $X^4$ is a cation of alkali and/or alkaline-earth metal, ammonium, alkyl ammonium, alkanol ammonium, glucoammonium;

Alkyl polyethylene glycol carboxylate of general formula: $R^5$-O(-$CH_2$-CH2-O-)$n^2CH_2$-$CO_2X^5$, wherein n2 is in the range from 1 to 15, and indicates the number of polyethylene glycol groups, $R^5$ is alkyl and/or alkenyl with hydrocarbon chain length from 6 to 22 carbon atoms, and $X^5$ is a cation of alkali and/or alkaline-earth metal, ammonium, alkyl ammonium, alkanol ammonium, glucoammonium;

Disubstituted salt of 2-sulphocarboxylic acid of general formula: $R^6$-CH(-$SO_3X^6$)-$CO_2X^6$, wherein $R^6$ is alkyl and/or alkenyl with hydrocarbon chain length from 4 to 20 carbon atoms, and $X^6$ is a cation of alkali and/or alkaline-earth metal, ammonium, alkyl ammonium, alkanol ammonium, glucoammonium;

Mono- or disubstituted amide salt of higher carboxylic acid and glutamic acid of general formula: $R^7$-C(O)-NH-CH(-

$CH_2$-$CH_2$-$CO_2X^7$)-$CO_2X^7$, wherein $R^7$ is alkyl and/or alkenyl with hydrocarbon chain length from 5 to 21 carbon atoms, and $X^7$ is a cation of alkali and/or alkaline-earth metal, ammonium, alkyl ammonium, alkanol ammonium, glucoammonium or hydrogen;

Amide salt of higher fatty acid and glycine of general formula: $R^8$-$C(O)$-$NH$-$CH_2$-$CO_2X^8$, wherein $R^8$ is alkyl and/or alkenyl with hydrocarbon chain length from 5 to 21 carbon atoms, and $X^8$ is a cation of alkali and/or alkaline-earth metal, ammonium, alkyl ammonium, alkanol ammonium, glucoammonium;

Amide salt of higher fatty acid and alanine of general formula: $R^9$-$C(O)$-$NH$-$CH(-CH_3)$-$CO_2X^9$, wherein $R^9$ is alkyl and/or alkenyl with hydrocarbon chain length from 5 to 21 carbon atoms, and $X^9$ is a cation of alkali and/or alkaline-earth metal, ammonium, alkyl ammonium, alkanol ammonium, glucoammonium ;

Amide salt of higher fatty acid and 2-aminomethyl ethane sulphonic acid of general formula: $R^{10}$-$C(O)$-$N(-CH_3)$-$CH_2$-$CH_2$-$SO_3X^{10}$, wherein $R^{10}$ is alkyl and/or alkenyl with hydrocarbon chain length from 5 to 21 carbon atoms, and $X^{10}$ is a cation of alkali and/or alkaline-earth metal, ammonium, alkyl ammonium, alkanol ammonium, glucoammonium;

Alkylpolyglucoside hydroxypropyl sulphonate of general formula: $R^{11}$-$O$-$[G]p^1$-$O$-$CH_2$-$CH(-OH)$-$CH_2$-$SO_3X^{11}$ , wherein $R^{11}$ is alkyl and/or alkenyl with hydrocarbon chain length from 6 to 22 carbon atoms, G is a saccharide fragment comprising 5 or 6 carbon atoms, $p^1$ is in the range from 1 to 4, and $X^{11}$ is a cation of alkali and/or alkaline-earth metal, ammonium, alkyl ammonium, alkanol ammonium, glucoammonium;

Alkylpolyglucoside carboxylate of general formula: $R^{12}$-$O$-$[G]p^2$-$O$-$CH_2$-$CO_2X^{12}$, wherein $R^{12}$ is alkyl and/or alkenyl with hydrocarbon chain length from 6 to 22 carbon atoms, G is a saccharide fragment comprising 5 or 6 carbon atoms, $p^2$ is in the range from 1 to 4, and $X^{12}$ is a cation of alkali and/or alkaline-earth metal, ammonium, alkyl ammonium, alkanol ammonium, glucoammonium;

Amide salt of higher fatty acid and threonine of general formula: $R^{13}$-$C(O)$-$NH$-$CH(-CH(-OH)-CH_3)$-$CO_2X^{13}$, wherein $R^{13}$ is alkyl and/or alkenyl with hydrocarbon chain length from 5 to 21 carbon atoms, and $X^{13}$ is a cation of alkali and/or alkaline-earth metal, ammonium, alkyl ammonium, alkanol ammonium, glucoammonium;

Amide salt of higher fatty acid and amino acid obtained by protein hydrolysis of the plant raw materials, of general formula: $R^{14}$-$C(O)$-$AAX^{14}$, wherein $R^{14}$ is alkyl and/or alkenyl with hydrocarbon chain length from 5 to 21 carbon atoms, AA is amino acid or peptide obtained from the plant protein hydrolysis (potential protein sources: apple, soybean, wheat, flax plant, etc.), and $X^{14}$ is a cation of alkali and/or alkaline-earth metal, ammonium, alkyl ammonium, alkanol ammonium, glucoammonium.

Amphoteric surfactants:

Disubstituted acyl amphodiacetate salt of general formula: $R^{15}$-$C(O)$-$NH$-$CH_2$-$CH_2$-$N(-CH_2$-$CO_2X^{15})$-$CH_2$-$CH_2$-$O$-$CH_2$-$CO_2X^{15}$, wherein $R^{15}$ is alkyl and/or alkenyl with hydrocarbon chain length from 5 to 21 carbon atoms, and $X^{15}$ is a cation of alkali and/or alkaline-earth metal, ammonium, alkyl ammonium, alkanol ammonium, glucoammonium;

Acyl amphoacetate salt of general formula: $R^{16}$-$C(O)$-$NH$-$CH_2$-$CH_2$-$N(-CH_2$-$CO_2X^{16})$-$CH_2$-$CH_2$-$OH$, wherein $R^{16}$ is alkyl and/or alkenyl with hydrocarbon chain length from 5 to 21 carbon atoms, and $X^{16}$ is a cation of alkali and/or alkaline-earth metal, ammonium, alkyl ammonium, alkanol ammonium, glucoammonium;

Alkyl amphoacetate salt of general formula: $R^{17}$-$C(=N-CH_2$-$CH_2$-$N((-CH_2$-$CH_2$-$OH)$-$CH_2$-$CO_2X^{17})-)$, wherein $R^{17}$ is alkyl and/or alkenyl with hydrocarbon chain length from 5 to 21 carbon atoms, and $X^{17}$ is a cation of alkali and/or alkaline-earth metal, ammonium, alkyl ammonium, alkanol ammonium, glucoammonium;

Acylamidoalkyl betaine of general formula: $R^{18}$-$C(O)$-$NH$-$R^{19}$-$N(-CH_3)_2)$-$CH_2$-$CO_2$ , wherein $R^{18}$ is alkyl and/or alkenyl with hydrocarbon chain length from 5 to 21 carbon atoms, $R^{19}$ is alkyl with hydrocarbon chain length from 1 to 4 carbon atoms;

Acylamidoalkylhydroxy sultaine of general formula: $R^{20}$-$C(O)$-$NH$-$R^{21}$-$N(-CH_3)_2$-$CH_2$-$CH(-OH)$-$CH_2$-$SO_3$, wherein $R^{20}$ is alkyl and/or alkenyl with hydrocarbon chain length from 5 to 21 carbon atoms, $R^{21}$ is alkyl with hydrocarbon chain length from 1 to 4 carbon atoms;

Acylamido alkylamine oxide of general formula: $R^{22}$-$C(O)$-$NH$-$R^{23}$-$N(-CH_3)_2$-$O$, wherein $R^{22}$ is alkyl and/or alkenyl with hydrocarbon chain length from 5 to 21 carbon atoms, $R^{23}$ is alkyl with hydrocarbon chain length from 1 to 4 carbon atoms;

Alkylbetaine of general formula: $R^{24}$-$N(-CH_3)_2)$-$CH_2$-$CO_2$, wherein $R^{24}$ is alkyl and/or alkenyl with hydrocarbon chain length from 5 to 21 carbon atoms;

Alkylhydroxy sultaine of general formula: $R^{25}$-$N(-CH_3)_2$-$CH_2$-$CH(-OH)$-$CH_2$-$SO_3$, wherein $R^{25}$ is alkyl and/or alkenyl with hydrocarbon chain length from 6 to 22 carbon atoms;

Alkylsultaine of general formula: $R^{26}$-$N(-CH_3)_2$-$CH_2$-$CH_2$-$CH_2$-$SO_3$, wherein $R^{26}$ is alkyl and/or alkenyl with hydrocarbon chain length from 6 to 22 carbon atoms;

Alkylamine oxide of general formula: $R^{27}$-N(-CH$_3$)$_2$-O, wherein $R^{27}$ is alkyl and/or alkenyl with hydrocarbon chain length from 6 to 22 carbon atoms.

Nonionic surfactants:

Alkylglucoside of general formula: $R^{28}$-O-[G]p$^3$, wherein $R2^8$ is alkyl and/or alkenyl with hydrocarbon chain length from 4 to 22 carbon atoms, G is saccharide fragment comprising 5 or 6 carbon atoms, p$^3$ is in the range from 1 to 4;

Alkylpolyethylene glycol of general formula: $R^{29}$-O(-CH$_2$-CH$_2$-O-)n$^3$H, wherein n$^3$ is in the range from 2 to 20, and indicates the number of polyethylene glycol groups, $R^{29}$ is alkyl and/or alkenyl with hydrocarbon chain length from 6 to 22 carbon atoms;

Alkylpolyethylene/propylene glycol of general formula: $R^{30}$-O(-CH$_2$-CH$_2$-O-)n$^4$(-CH(-CH$_3$)-CH$_2$-O-)n$^5$H, wherein n$^4$ is in the range from 2 to 20, and indicates the number of polyethylene glycol groups, n$^5$ is in the range from 2 to 20, and indicates the number of polypropylene glycol groups, $R^{30}$ is alkyl and/or alkenyl with hydrocarbon chain length from 6 to 22 carbon atoms;

Dialky lpolyethylene glycol of general formula: $R^{31}$-O(-CH$_2$-CH$_2$-O-)n$^6$R$^{32}$, wherein n$^6$ is in the range from 2 to 20, and indicates the number of polyethylene glycol groups, $R^{31}$ is alkyl and/or alkenyl with hydrocarbon chain length from 6 to 22 carbon atoms, $R^{32}$ is alkyl and/or alkenyl with hydrocarbon chain length from 1 to 12 carbon atoms;

Dialkyl polethylene/propylene glycol of general formula: $R^{33}$-O(-CH$_2$-CH$_2$-O-)n$^7$(-CH(-CH$_3$)-CH$_2$-O-)n$^8$-R$^{34}$, wherein n$^7$ is in the range from 2 to 20, and indicates the number of polyethylene glycol groups, n$^8$ is in the range from 2 to 20, and indicates the number of polypropylene glycol groups, $R^{33}$ is alkyl and/or alkenyl with hydrocarbon chain length from 6 to 22 carbon atoms, $R^{34}$ is alkyl and/or alkenyl with hydrocarbon chain length from 1 to 12 carbon atoms.

Dispersing medium for polysaccharide/solvent:

Organic alcohol of general formula: $R^{35}$(-OH)s$^1$, wherein $R^{35}$ is alkyl with hydrocarbon chain length from 3 to 12 carbon atoms, s$^1$ is in the range from 1 to 12, and indicates the number of hydroxylic groups located and hydrocarbon radical in random order with respect to each other;

Alkylpolypropylene glycol of general formula: H(-CH(-CH$^3$)-CH$^2$-O-)n$^9$R$^{36}$, wherein n$^9$ is in the range from 2 to 10, and indicates the number of polypropylene glycol groups, $R^{36}$ is alkyl with hydrocarbon chain length from 1 to 10 carbon atoms.

pH adjusting agents:

Organic acids of general formula: $R^{37}$(-OH)s$^2$(-COOH)m$^1$, wherein $R^{37}$ is alkyl with hydrocarbon chain length from 1 to 12 carbon atoms, s$^2$ is in the range from 1 to 12, and indicates the number of hydroxylic groups located and hydrocarbon radical in random order with respect to each other, m$^1$ is in the range from 1 to 4, and indicates the number of carboxylic groups located and hydrocarbon radical in random order with respect to each other;

Solutions of alkali and/or alkaline-earth metal hydroxides, ammonium hydroxide, primary and tertiary alkyl amines, primary and tertiary alkanol amines, primary and tertiary glucamines, basic amino acids, disodium citrate, trisodium citrate.

Chelating agent:

Trisodium methyl glycine-diacetate, tetrasodium of glutamine-diacetate, trisodium of ethylenediamine-(N,N)-disuccinate;

Phosphonic acid esters of general formula RP(O)(OR'$_1$)n(OH)$_{2-n}$, wherein R, R' are organic radicals, in particular, alkyl, alkenyl or aryl radical; said esters may be primary (n=1, acidic phosphonates) or secondary (n=2, full phosphonate) depending on hydroxylic groups;

Organic acids, and salts formed by alkali metals, ammonium, alkyl ammonium, glucoammonium and said organic acids: citric acid, malic acid, tartaric acid, glutaric acid, adipic acid, glucuronic acid, galacturonic acid, galactaric acid, gluconic acid, phytic acid, polyitaconic acid, polyacrylic acid, polymetacrylic acid, of acrylic/metacrylic and maleinic acid co-polymer, and also organic acids of general formula $R_3^8(-OH)s^3(-COOH)m^2$, wherein $R^{38}$ is alkyl with hydrocarbon chain length from 1 to 12 carbon atoms, $s^3$ is in the range from 1 to 12, and indicates the number of hydroxylic groups located and hydrocarbon radical in random order with respect to each other, $m^2$ is in the range from 1 to 4, and indicates the number of carboxylic groups located in hydrocarbon radical in random order with respect to each other.

Inhibitors of contaminant redeposition:

Polysaccharide derivatives: sodium carboxymethyl polysaccharide, hydroxyalkyl polysaccharide, alkyl polysaccharide;
Polyvinyl pyrrolidone and the derivatives thereof; polyvinyl pyrrolidone and vinyl imidazole co-polymers;
[0047] Water-soluble salts of polyacrylic acid, polymetacrylic acid, of acrylic/metacrylic and maleinic acid co-polymer.

Antifoaming agents:

Higher carboxylic acids of general formula: $R^{39}-CO_2H$, wherein $R^{39}$ is alkyl and/or alkenyl with hydrocarbon chain length from 5 to 21 carbon atoms;
Higher carboxylic alcohols of general formula: $R^{40}-COH$, wherein $R^{40}$ is alkyl and/or alkenyl with hydrocarbon chain length from 5 to 21 carbon atoms;
Ethers of higher carboxylic alcohols of general formula: $R^{41}-O-R^{42}$, wherein $R^{41}$, $R^{42}$ are alkyl and/or alkenyl with hydrocarbon chain length from 4 to 22 carbon atoms;
Bis-amides of alkyldiamines and higher carboxylic acids of general formula: $R^{43}-C(O)-NH-R^{44}-NH-C(O)-R^{45}$, wherein $R^{43}$, $R^{45}$ are alkyl and/or alkenyl with hydrocarbon chain length from 5 to 21 carbon atoms, and $R^{44}$ is alkyl with hydrocarbon chain length from 1 to 12 carbon atoms;

Preservatives:

Organic acids and salts formed by said acids and of alkali metals, alkaline-earth metals, ammonium, alkyl ammonium, alkanol ammonium, glucoammonium: benzoic acid, sorbic acid, 4-methoxybenzoic acid, salicilic acid, undecanoic acid;
Organic alcohols and phenols: phenoxyethanol, benzyl alcohol, caprylyl glycol, ethylhexyl glycerol phenethyl alcohol, 3-methyl-4-isopropylphenol, 2,4-dichlorobenzyl alcohol;
Wide-spectrum biocides: benzisothiazolinone, dodecylpropylene triamine, methylisothiazolinone;

Fungicides: sodium pyrithione, climbazole.

Enzymes: protease (ficine, bacillolysin, subtilisin), alpha-amylase, pectate lyase, mannanase, mannosidase, cellulase, amine oxidase, ferruloyl esterase, beta-glucanase, tannase, alpha-glucosidase, beta-glucosidase, alpha-galactosidase, beta-galactosidase, laccase, manganese peroxydase, licheninase, xylanase, and other commercially available enzymes, which are used in laundry washing detergents, dishwashing detergents, cleaners for floors, glass, and multi-surface cleaners.
Bleaching agents based on oxygenated compounds: hydrogen peroxide, calcium peroxide, carbamide peroxide, ε-phthalimidocaproic acid, and other commercially available components.
Perfume agents containing essentials oils, or neat essential oils, or essential oils mixes prepared at various proportions: orange, bergamot, lemon, lime, tangerine, grape fruit, neroli, rose wood, yuja, lemongrass, lavender, sage, thyme, melissa, mint, tea tree, eucalypt, cedar, sandal, black pepper, pink pepper, cinnamon, cardamon, coriander, jasmine, rose, peony, cyan chamomile, or other.

EXPERIMENTS

The examples in this specification are not considered as limiting the present invention, and are included merely for illustrative purpose and to support the achievement of the expected technical results. These examples are among many experimental findings obtained by the Authors of invention, which prove the efficacy of formulations under the scope of invention.

**Example 1.**

Shikimic acid was tested as an ingredient of various household chemical formulations. For this purpose liquid detergent formulation was prepared (Formulation No1), specifically, all-purpose laundry washing detergent for white and color clothes under the scope of invention (Table 1).

Table 1. The composition of liquid laundry washing detergent formulation comprising shikimic acid

| No | Ingredient | Level, %wt. |
|---|---|---|
| 1 | Purified water | To 100.00 |
| 2 | Alkylglucoside of general formula: $R^{28}$-O-[G]$p^3$, wherein $R^{28}$ is alkyl and/or alkenyl with hydrocarbon chain length from 4 to 22 carbon atoms, G is a saccharide fragment comprising 5 or 6 carbon atoms, $p^3$ is in the range from 1 to 4, in particular, C10-16 alkylpolyglucoside и C8-10 alkylpolyglucoside | 1.5-10.00 |
| 3 | Acylamidoalkyl betaine of general formula: $R^{18}$-C(O)-NH-$R^{19}$-N(-CH$_3$)$_2$)-CH$_2$-CO$_2$, wherein $R^{18}$ is alkyl and/or alkenyl with hydrocarbon chain length from 5 to 21 carbon atoms, $R^{19}$ is alkyl with hydrocarbon chain length from is alkyl with hydrocarbon chain length from 1 to 4 carbon atoms | 0.5-5.00 |
| 4 | Compound mixture of ethoxylated alcohols and fatty acids at pH 4.5 (method EN 1262), cloud point at 44-53°C (method EN 1890), specific gravity 0.95 g/mL (method DIN 51757) and viscosity 70mPa*s (method EM 12092, 23°C, Brookfield, 60 rpm) | 1.5-7.50 |
| 5 | Helating agent methylglycinediacetate (MGDA) | 0.1-1.00 |
| 6 | Chelating corrosion inhibitor based on inulin derivative | 0.1-1.00 |
| 7 | Natural glycerol | 1.0-5.00 |
| 8 | Trisodium citrate dihydrate | 0.5-3.00 |
| 9 | Sodium chloride | 1.0-5.00 |
| 10 | Cotton seed extract | 0.005-0.50 |
| 11 | Preservative | 0.005-0.75 |
| 12 | Sodium hydroxide or potassium hydroxide | 0.005-0.50 |
| 13 | Additives, if necessary | 0.005-25.00 |
| 14 | Shikimic acid | 0.001-0.50 |

Prepared liquid laundry washing detergent and stain removing formulation showed effective cleaning and unpleasant odor removing from fabrics, in particular, those made of cotton, synthetic, membrane, linen at any tap water hardness in the range from 0 to 15 dH and at any washing temperature in the range from +15°C to +60°C. The formulation does not cause the fabric discoloration and dye washout, preserves the initial appearance of the clothes, does not leave streaks, is completely washed off from the fabric surface, and suitable for laundry of the childnren's clothes and for the persons with sensitive skin on the hands.

**Example 2.**

Shikimic acid was investigated as an ingredient of various household chemical formulations. For this purpose dry detergent formulation was prepared (Formulation No2), specifically, powder laundry washing detergent for color clothes and stain removing under the scope of invention (Table 2).

Table 2. The composition of powder laundry washing detergent formulation comprising shikimic acid for color clothes.

| No | Ingredient | Level, % wt. |
|---|---|---|
| 1 | Purified water | 0.5-7.5 |
| 2 | Alkylpolyethylene glycol of general formula: $R^{29}$-O(-$CH_2$-$CH_2$-O-)$n^3$H, wherein $n^3$ is in the range from 2 to 20 and indicates the number of polyethylene glycol groups, $R^{29}$ is alkyl and/or alkenyl with hydrocarbon chain length from 6 to 22 carbon atoms | 2.5-10.0 |
| 3 | Alkyl carboxylate of general formula $R^1$-O(-$CH_2$-$CH_2$-O)$n^1$ ($SO_3$) $n^2$ $X^1$, wherein $n^1$ is in the range from 0 to 10 and indicates the number of polyethylene groups, $R^1$ is alkyl and/or alkenyl with hydrocarbon chain length from 5 to 22 carbon atoms, $n^2$ is in the range from 0 to 1 and indicates the number of sulphate groups, $X^1$ is a cation of alkali and/or alkaline-earth metal, ammonium, alkyl ammonium, alkanol ammonium, glucoammonium, basic amino acid | 1.0-10.0 |
| 4 | Sodium carbonate | 10.0-40.0 |
| 5 | Sodium sulphate | 10.0-50.0 |
| 6 | Sodium silicate | 0.5-10.0 |
| 7 | Sodium aluminosilicate as zeolite | 5.0-20.0 |
| 8 | Sodium aluminosilicate amorphous | 1.0-10.0 |
| 9 | TAED | 0.1-1.5 |
| 10 | Lipase, mannanase, cellulase, amylase | 0.001-0.25 |
| 11 | Sodium carboxymethylcellulose | 0.15-2.50 |
| 12 | Carboxymethylcellulose | 0.5-2.00 |
| 13 | Cotton seed extract | 0.005-0.50 |
| 14 | Additives, if necessary | 2.0-25.0 |
| 15 | Shikimic acid | 0.001-0.50 |

Prepared powder laundry washing detergent formulation for color clothes and stain removing showed effective cleaning and unpleasant odor removing from fabrics, in particular, those made of cotton, synthetic, membrane, linen at any tap water hardness in the range from 0 to 15 dH and at any washing temperature in the range from +15°C to +60°C. The formulation does not cause the fabric discoloration and dye washout, preserves the initial appearance of the clothes, does not leave streaks, is completely washed off from the fabric surface, and suitable for laundry of the children's clothes and for the persons with sensitive skin on the hands.

**Example 3.**

Shikimic acid was investigated as an ingredient of various household chemical formulations. For this purpose dry detergent formulation was prepared (Formulation No3), specifically, powder laundry washing detergent for white clothes and stain removing under the scope of invention (Table 3).

Table 3. The composition of powder laundry washing detergent formulation for white clothes comprising shikimic acid for white clothes

| No | Ingredient | Level, % wt. |
|---|---|---|
| 1 | Purified water | 2.0-7.5 |
| 2 | Alkylpolyethylene glycol of general formula: $R^{29}$-O(-$CH_2$-$CH_2$-O-)$n^3$H, wherein $n^3$ is in the range from 2 to 20 and indicates the number of polyethylene glycol groups, $R^{29}$ is alkyl and/or alkenyl with hydrocarbon chain length from 6 to 22 carbon atoms | 1.0-10.0 |

(continued)

| No | Ingredient | Level, % wt. |
|---|---|---|
| 3 | Alkyl carboxylate of general formula $R^1$-O(-$CH_2$-$CH_2$-O)n1 ($SO_3$) $n^2$ $X^1$, wherein n1 is in the range from 0 to 10 and indicates the number of polyethylene groups, $R^1$ is alkyl and/or alkenyl with hydrocarbon chain length from 5 to 22 carbon atoms, $n^2$ is in the range from 0 to 1 and indicates the number of sulphate groups, $X^1$ is a cation of alkali and/or alkaline-earth metal, ammonium, alkyl ammonium, alkanol ammonium, glucoammonium, basic amino acid | 1.0-10.0 |
| 4 | Sodium carbonate | 10.0-40.0 |
| 5 | Sodium carbonate peroxide | 5.0-20.0 |
| 6 | Sodium sulphate | 4.0-30.0 |
| 7 | Sodium silicate | 0.5-10.0 |
| 8 | Sodium aluminosilicate as zeolite | 5.0-20.0 |
| 9 | Sodium aluminosilicate amorphous | 1.0-10.0 |
| 10 | Tetraacetyl ethylene diamine (TAED) | 0.5-5.0 |
| 11 | Lipase, mannanase, cellulase, amylase | 0.001-0.25 |
| 12 | Sodium carboxymethylcellulose | 0.15-2.50 |
| 13 | Carboxymethylcellulose | 0.5-2.00 |
| 14 | Cotton seed extract | 0.005-0.50 |
| 15 | Additives, if necessary | 2.0-25.0 |
| 16 | Shikimic acid | 0.001-0.50 |

Prepared powder laundry washing detergent and stain removing formulation for white clothes showed effective cleaning and unpleasant odor removing from fabrics, in particular, made of cotton, synthetic, membrane, linen at any tap water hardness in the range from 0 to 15 dH and at any washing temperature in the range from +15°C to +60°C. The formulation does not cause the fabric discoloration and dye washout, preserves the initial appearance of the clothes, does not leave streaks, is completely washed off from the fabric surface, and suitable for laundry of the children's clothes and for persons with sensitive skin on the hands.

**Example 4.**

Shikimic acid was investigated as an ingredient of various household chemical formulations. For this purpose liquid detergent formulation was prepared (Formulation No4), specifically, laundry washing conditioner for clothes refreshing under the scope of invention (Table 4).

Table 4. The composition of laundry conditioner formulation comprising shikimic acid

| No | Ingredient | Level, %wt. |
|---|---|---|
| 1 | Purified water | to 100.00 |
| 2 | Calcium lactate | 0.005-0.50 |
| 3 | Palm esterquat | 2.0-25.00 |
| 4 | Natural glycerol | 1.0-5.00 |
| 5 | Cotton seed extract | 0.005-0.50 |
| 6 | Preservative | 0.005-0.75 |
| 7 | Shikimic acid | 0.001-0.50 |

Prepared laundry conditioner provides softening and easier ironing of various fabrics, in particular, made of cotton, synthetic, membrane, linen at any tap water hardness in the range from 0 to 15 dH and at any washing temperature in the range from +15°C to +60°C. The formulation does not cause the fabric discoloration and dye washout does not leave streaks, and is suitable for laundry of the children's clothes and for persons with sensitive skin on the hands.

**Example 5.**

Shikimic acid was investigated as an ingredient of various household chemical formulations. For this purpose liquid detergent formulation was prepared (Formulation No5), specifically, a dishwashing cleaner under the scope of invention (Table 5).

Table 5. The composition of dishwashing cleaner formulation comprising the claimed composition.

| No | Ingredient | Level, % wt. |
|---|---|---|
| 1 | Purified water | to 100.00 |
| 2 | Alkylglucoside of general formula: $R^{28}$-O-[G]$p^3$, wherein $R^{28}$ is alkyl and/or alkenyl with hydrocarbon chain length from 4 to 22 carbon atoms, G is a saccharide fragment comprising 5 or 6 carbon atoms, $p^3$ is in the range from 1 to 4, specifically, C10-16 alkylpolyglucoside И C8-10 alkylpolyglucoside | 1.5-10.00 |
| 3 | Acylamidoalkyl betaine of general formula: $R^{18}$-C(O)-NH-$R^{19}$-N(-CH$_3$)$_2$)-CH$_2$-CO$_2$, wherein $R^{18}$ is alkyl and/or alkenyl with hydrocarbon chain length from 5 to 21 carbon atoms, $R^{19}$ is alkyl with hydrocarbon chain length from 1 to 4 carbon atoms | 0.5-5.00 |
| 4 | Alkyl carboxylate of general formula $R^1$-O(-CH$_2$-CH$_2$-O)n1 (SO$_3$) $n^2$ $X^1$, wherein n1 is in the range from 0 to 10 and indicates the number of polyethylene groups, $R^1$ is alkyl and/or alkenyl with hydrocarbon chain length from 5 to 22 carbon atoms, $n^2$ is in the range from 0 to 1 and indicates the number of sulphate groups, $X^1$ is a cation of alkali and/or alkaline-earth metal, ammonium, alkyl ammonium, alkanol ammonium, glucoammonium | 1.0-10.0 |
| 5 | Cotton seed extract | 0.005-0.5 |
| 6 | Aqueous solution of citric acid salt and citrate | 0.001-0.5 |
| 7 | Benzyl alcohol | 0.1-1.0 |
| 8 | Sodium cocoyl gutamate | 0.25-5 |
| 9 | Sodium chloride | 0.01-5 |
| 10 | Trisodium citrate dihydrate | 0.01-5 |
| 11 | Sodium cocoamphoacetate | 0.25-5 |
| 12 | Shikimic acid | 0.001-0.50 |
| 13 | pH adjusting agent (citric acid) | 0.005-0.50 |
| 14 | Natural glycerol | 1.0-5.0 |

Prepared dishwashing cleaner formulation showed effective dish cleaning from contaminants of protein, carbohydrate and lipid origins and also from pigments entering the composition of food colorants and natural substances, in particular, carotenoids, flavonoids, anthocyans, etc.; in addition, the formulation contributes to neutralization of unpleasant odors at any tap water hardness in the range from 0 to 15 dH and at any washing temperature in the range from +15°C to +60°C. The formulation does not cause the skin irritation and is suitable for the persons with sensitive skin on the hands.

**Example 6.**

The components to be included into the composition of invention were investigated as the ingredients of various household chemical formulations. For this purpose liquid cleaning formulation (Formulation No 6) was prepared, specifically, multi-surface cleaner under the scope of invention (Table 6).

Table 6. The composition of multi-surface cleaner comprising the claimed composition

| No | Ingredient | Level, % wt. |
|---|---|---|
| 1 | Purified water | to 100 |
| 2 | Salts of higher carboxylic acids of general formula: $R^1-CO_2X^1$, wherein $R^1$ is alkyl and/or alkenyl with hydrocarbon chain length from 5 to 21 carbon atoms, a $X^1$ is a cation of alkali and/or alkaline-earth metal, ammonium, alkyl ammonium, alkanol ammonium, glucoammonium, basic amino acid | 1-15 |
| 3 | Alkylglucoside of general formula: $R^{28}-O-[G]p^3$, wherein $R^{28}$ is alkyl and/or alkenyl with hydrocarbon chain length from 4 to 22 carbon atoms, G is a saccharide fragment comprising 5 or 6 carbon atoms, $p^3$ is in the range from 1 to 4, specifically | 2-15 |
| 4 | Biolipase in aqueous glycerol solution of enzyme Lipex Evity® 200L | 0.005-0.01 |
| 5 | Chelating agent | 0.1-0.8 |
| 6 | Cotton seed extract | 0.05-0.15 |
| 7 | Aqueous solution of citric acid and silver citrate | 0.001-0.1 |
| 8 | Citric acid monohydrate | 0.01-0.3 |
| 9 | Preservative | 0.2-0.8 |
| 10 | Shikimic acid | 0.001-0.50 |

Prepared multi-surface cleaner formulation showed effective splitting of low melting saturated and/or unsaturated acids and respective monoacyl-, diacyl-, and triacylglycerides having various number of links with sp2-hybridization, in cis- or trans-configuration, on various surfaces including metallic, polymeric, glass and wooden surfaces. The formulation does not cause corrosion of the cleaned surfaces, in particular, no limescale deposits, and is stable when stored for 24 months (observation period).

**Example 7.**

Detergent efficacy of shikimic acid as an ingredient of liquid all-purpose laundry washing detergent was tested in laboratory investigation. Liquid all-purpose laundry washing detergent for white and color clothes shown in Table 1 was taken as a reference and was supplemented by other ingredients.

The test procedure was based on established recommendations of European Association A.I.S.E. [A.I.S.E. Laundry Detergent Testing Guidelines Minimum requirements for comparative detergents testing https://www.aise.eu/documents/document/20180625164030-laundry_detergent_testing_guidelines_v_5_2_june_2018_.pdf] and was used to assess detergent efficacy based on stain removal index (SRI) И values L, a, b. This Guidelines has been prepared by Committee D-12 - Soap and other detergent analysis committee under American Society for Testing and Materials (ASTM) and was published in 2014. The test allows to assess the composition efficacy based on degree of removal of fresh and heavy stains of various nature from cotton and synthetic fabrics under conditions simulating real washing conditions. Comparative assessment of the stains was carried out in accordance with recommendations of European Association A.I.S.E. based on the degree of washing off the stain (SRI) with the use of values L, a, b for calculation by the equation ASTM D 4265 Standard guide for evaluating stain removal performance in home laundering [DOI: 10.1520/D4265-14]. Results of investigation are summed up in Table 7. The higher SRI value the more the fabric cleanliness corresponds to initial unstained fabric.

$$SRI=100-[(L_c-L_w)^2+(a_c-a_w)^2+(b_c-b_w)^2]^{1/2}$$

Wherein: L - reflection power, a - red/green ratio, b - yellow/blue ratio, c - initial unstained fabric, w - washed stained fabric.

The laundry was carried out in washing machine Bosch WAB 24272 CE. Common recommendations for EU territory and in Eurasian region were used to select the test conditions, in particular, temperature regimen 40°C, water hardness 10 dH and standard washing mode (mixed fabrics, 1 hour). Liquid all-purpose laundry washing detergent for white and color clothes was used at concentration 4.5 g/L per standard loading (2-4 kg) of washing machine. Three replicates were used (n=3), for each sample the error was 0,10% or less.

In investigation all-purpose laundry gel detergent (formulation No 1) supplemented with shikimic acid in amount of 0.001%, 0.01% and 0,1% and reference sample without shikimic acid were used. For the test purposes the standardized stubborn biostains of various origin were used (CFT Netherlands): C-H244 PDE Stain - a spot reflecting the stain forming on the body in the course of normal vital activities (sweat, sebum, DNA, etc.), and also C-S-17 Fluid makeup - a spot typical for makeup stain. Selected stubborn biostains allowed to assess both detergent formulation efficacy as a whole, and shikimic acid efficacy against separate types of stain. Humans face both types of stain in everyday life, and this contact results in declining clothes cleanliness, appearance of unpleasant odor and lower level of hygiene as a whole. In particular, enhanced sebum secretion is associated with the risk of acne, and accumulation of natural secretions can serve as an environment favouring the bacterial pathogen development [Li X. et al. A review of the role of sebum in the mechanism of acne pathogenesis, 2017].

SRI is a quantitative stain and spot removal index expressed in arbitrary units. The values are based on whiteness L and color (a, b) indices are calculated mathematically using special equation from standard ASTM D 4265-14. The method has many advantages, in particular, high accuracy, reproducibility, low error (< 5%), consideration of the clothes color index and alteration thereof during washing process, various fabric types and is the closest to real visual perception, and also balances the effects of optic dyes commonly used in the laundry washing detergent formulations. The difference by 2 or more SRI units is effective and statistically significant. From practical experience, 1 SRI unit is equal to 5% detergent efficacy and makes significant contribution to the formulation.

Results.

Based on assessment of efficacy and elimination rate of stubborn biostains of various nature, shikimic acid used as an ingredient of liquid all-purpose laundry washing detergent for white and color clothes demonstrated marked stain-removing effect against complex biostain without loss of efficacy. At the end of investigation major changes of the assessed efficacy index of the stubborn biostain removal were observed. According to the dynamics of that index, addition of shikimic acid in amount from 0.001% to 0,1% allowed to considerably increase the degree of removal of selected spot and to produce hygienic effect on the clothes. Addition of 0.1% shikimic acid allowed to increase SRI by 2.0 units in relation to stain comprising sweat with DNA ($p < 0.05$) and by 14.47 units in relation to makeup stain ($p < 0.01$) compared to the reference samples without shikimic acid. The reference sample free from the composition of the invention did not demonstrate very high level of selected biostains removal, thus indicating inadequate surfactant efficacy in terms of stain removal in the course of washing (Table 7).

Table 7. Investigation of efficacy of removal of standardized CFT stains with the use of laundry washing detergent gel comprising shikimic acid.

| Test sample | SRI | |
|---|---|---|
| | PDE Stain | Makeup Stain |
| SA 0% | 41.70 | 69.70 |
| SA 0.001% | 40.70 | 83.74 |
| SA 0.01% | 42.90 | 80.05 |
| SA 0,1% | 43.70 | 84.17 |
| SA - shikimic acid | | |

Based on the above data one can conclude that shikimic acid considerably enhances the efficacy of washing off the makeup - the difference ranged from 10.35 to 14.47 compared to reference sample. Furthermore, the gel comprising shikimic acid is potentially suitable for removing the stain comprising sweat, sebum, and DNA thus providing hygiene as a whole, and, in particular, decreases the chance of unpleasant odor formation, the difference compared to the reference sample was up to 2 units. Therefore, addition of shikimic acid in the amount from 0.001% to 0.5% to the formulation improves hygienic and cleaning effects of the laundry washing detergents while does not affect physical-chemical properties of the product.

**Example 8.**

Stain-removing efficacy of shikimic acid as an ingredient of liquid all-purpose laundry washing detergent was tested in laboratory investigation. Liquid all-purpose laundry washing detergent for white and color clothes shown in Table 1 was taken as a reference and was supplemented by other ingredients.

The test procedure was based on established recommendations of European Association A.I. S.E. and is described in details in Example 7. All-purpose laundry gel detergent with stain-removing effect (formulation No 1) supplemented with 0.25% shikimic acid was compared to reference formulation without shikimic acid. For the test purposes stubborn biostains of various nature including those containing melanoidins and/or organic phenolic compounds, vegetable oil and starch were used (Table 8). Selected stubborn biostains allowed to assess both stain-removing efficacy of formulation as a whole, and shikimic acid compatibility with enzymes-ingredients of the formulation, in particular amylase and lipase.

Table 8. Tested biospots and biostains from A.I.S.E.

| Name | Composition | Type |
|---|---|---|
| EMPA 106 | Mineral oil /soot | Pigment-oil, dispersible |
| EMPA 118 | Sebum / pigment | Pigment-oil, dispersible |
| EMPA 116 | Blood / milk/ ink | Protein |
| CFT CS-08 | Grass | Mixed: protein and bleachable * |
| CFT CS-44 | Chocolate drink | Mixed* |
| CFT CBC-01 | Tea | Bleachable* |
| EMPA 114 | Red wine | Bleachable* |
| CFT CS-12 | Black currant juice | Bleachable* |
| CFT CBC-02 | Coffee | Bleachable* |
| *comprise ordinary or complex melanoidins and/or phenolic organic compounds ||| 

Standard stained samples (55x45 mm in size) were fixed on dense white cotton tissue, which was placed on the flat table surface; then 2-3 mL of distilled water were applied on the stains followed by 2 mL of pre-spotter and uniformly distributed on the stain surface (5 circular motions clockwise + 5 circular motions counterclockwise, simulating usual washing process) using stiff plastic brush. The washing operation was repeated on each stain 10 minutes later. Then treated stained samples were transferred into the washing machine and washed with the use of 45 g of liquid all-purpose laundry washing detergent described in Example 1. For comparative assessment of washing results the same stained samples were similarly washed without pretreatment, and also commercial household chemicals were used.

Results.

Based on assessment of efficacy and elimination rate of stubborn stains, 0.25% shikimic acid added to all-purpose concentrated gel with stain-removing effect demonstrated marked stain-removing effect against stubborn stains compared to reference formulation without shikimic acid of the invention. These findings indicate the shikimic acid compatibility with particular enzymes and basic ingredients of formulation of the invention (Example 1), expand the potential use of shikimic acid as an ingredient of household stain-removing chemicals and offer new solution for hygienic cleaning and top-quality stain removing.

At the end of investigation major changes of the assessed efficacy index of the stubborn biostain removal from cotton tissue were observed. According to the dynamics of that index, addition of 0.25% shikimic acid considerably improved the removal of complex stain by 43.8 SRI units ($p < 0.05$). The use of shikimic acid allowed to remove the stains comprising oil, sebum blood, starch, coffee, tea, vine, grass and chocolate drink thus demonstrating compatibility of lipase, amylase and shikimic acid present together in the laundry gel formulation. Reference formulation without shikimic acid of the invention did not demonstrate very good stain and spot removing results suggesting inadequate surfactant efficacy in respect to stain and spot removing during the washing process and need for shikimic acid addition (Table 9).

Table 9. Assessment of stain and spot removing efficacy

| Test sample | Active ingredients | SRI | | | | | | | | | Total SRI |
|---|---|---|---|---|---|---|---|---|---|---|---|
| | | EMPA 106 | EMPA 118 | EMPA 116 | EMPA 114 | CFT CS-08/ EMPA 164 | CFT CS-44 | CFT CS-12 | CFT CBC-01/ WFK 10J | CFT CBC-02 | |
| Laundry gel sample | - | 72.3 | 89.9 | 76.3 | 87.9 | 80.8 | 79.8 | 72.5 | 85.8 | 84.1 | 729.4 |
| laundry gel sample | Shikimic acid 0.25% | 85.1** | 92.5* | 84.9** | 90.8* | 90.9** | 84.2** | 75.1* | 86.4 | 84.5* | 773.2** |

Shikimic acid can inhibit amylase, which is used for starch stain removing [Cao J. et al. Elucidation of alpha-amylase inhibition by natural shikimic acid derivates regarding the infrequent uncompetitive inhibition mode and structure-activity relationship, 2023], and lipase, which splits fatty stain [Raw at G. et al. An interactive study of influential parameters for shikimic acid production using statistical approach, scale up and its inhibitory action on different lipases, 2013]. However, the laundry gel formulation supplemented with shikimic acid demonstrated stability in pH range from 8 to 8.5 and provided high stain-removing effect. The Authors of the invention unexpectedly found synergetic effect of the use of shikimic acid in the composition of various household chemicals as a stabilizer and viscosity regulator. The technical result of the use of shikimic acid consists in effective cleaning of various fabrics and making the closes clean and crystal white.

**Example 9.**

Comparative investigation was carried out to assess the efficacy of limescale deposit removing with the use of multi-surface cleaning spray comprising 0.25% shikimic acid (Formulation No 5), the detergents based on natural organic acids (5-15% of citric acid and <5% of malic acid), and the detergent based on organic synthetic acid (<5% of oxalic acid). The tests were carried out in accordance with OST 2309-005-00209645-94 "Test method for cleaning efficacy of the cleaners for painted and polymeric surfaces" and STO 70864601-9.2-2023 "Test method by Hampshire for dispersing (chelating) power".

According to investigation results, shikimic acid added to the multi-surface cleaning spray demonstrated the highest dispersing (chelating) power, which characterizes the efficacy of limescale deposit removing from the various surfaces. The achieved effect was 2-4-fold compared to usual organic acids used at higher concentrations. The use of shikimic acid allows to make the formulation more effective and safe for the skin on the hands.

Table 10. Results of assessment of cleaing efficacy

| Test sample | Active ingredients | Cleaning efficacy | Dispersing power |
|---|---|---|---|
| Household spray sample | Shikimic acid 0,25% | 100 | 15.1 |
| Household spray sample | Citric acid 5-15% Malic acid <5% | 100 | 4.3 |
| Household spray sample | Oxalic acid <5% | 100 | 8.2 |

**Example 10.**

The laundry gel (Example 1, formulation 1) was used as the reference for assessment of stability of laundry gels supplemented by shikimic acid in amount of 0.001%, 0.01%, 0,1%, and 0.5%.

The following stability parameters were used:

1. Dynamic viscosity - DV2T Brookfield viscosimeter was used for the measurements;
2. pH - measured by IPL 103 multitest pH-meter;

3. Visual evaluation of the appearance (for the presence of sediment or layering).

Viscosity was measured on 3 occasions - 25 and 32 days after the start of investigation; pH was measured every 6-12 days during the 32-day period. The results of investigation are summed up below. Each dynamic viscosity value is a mean arithmetic calculated for three replicates. All samples designated as No 1 were stored at room temperature (+23°C), samples No 2 were stored in thermostat (at +40°C). Normal viscosity of the laundry gel is - 250-700 Pa*s, normal pH 7.7-8.4.

Table 11. Test for dynamic viscosity of the laundry gel

| Day | Reference No 1 | Reference No 12 | SA 0.001% No 1 | SA 0.001% No 2 | SA 0.01% No 1 | SA 0.01% No 2 | SA 0,1% No 1 | SA 0,1% No 2 | SA 0.5% No 1 | SA 0.5% No2 |
|---|---|---|---|---|---|---|---|---|---|---|
| 0 | 660 | 660 | 656 | 656 | 700 | 700 | 1050 | 1050 | 300 | 300 |
| 25 | 694 | 700 | 727 | 755 | 791 | 847 | 1085 | 1037 | 288 | 296 |
| 32 | 669 | 704 | 779 | 680 | 793 | 769 | 1073 | 1042 | 273 | 277 |
| SA - shikimic acid concentration. Units -Pa*s. | | | | | | | | | | |

Table 12. Test for pH of the laundry gel.

| Day | Reference No 1 | Reference No 12 | SA 0.001% No 1 | SA 0.001% No2 | SA 0.01% No 1 | SA 0.01% No 2 | SA 0,1% No 1 | SA 0,1% No 2 | SA 0.5% No 1 | SA 0.5% No2 |
|---|---|---|---|---|---|---|---|---|---|---|
| 0 | 8.04 | 8.04 | 8.02 | 8.02 | 8.02 | 8.02 | 7.78 | 7.78 | 6.95 | 6.95 |
| 7 | 7.96 | 7.93 | 7.97 | 7.97 | 7.92 | 7.93 | 7.61 | 7.61 | 6.77 | 6.74 |
| 19 | 8.09 | 8.03 | 8.07 | 8.03 | 8.03 | 8.05 | 7.72 | 7.71 | 6.88 | 6.84 |
| 25 | 8.10 | 8.11 | 8.10 | 8.10 | 8.04 | 8.08 | 7.74 | 7.76 | 6.94 | 6.91 |
| 32 | 8.12 | 8.12 | 8.08 | 8.12 | 8.10 | 8.09 | 7.79 | 7.80 | 7.02 | 6.97 |
| SA - shikimic acid concentration. | | | | | | | | | | |

During the course of experiment no discoloration, cloudiness or layering of the sample were observed. The measured pH values suggest that the gels comprising shikimic acid in amounts ranging from 0.001% to 0.25% are stable for 32 days. The laundry gel comprising 0.5% shikimic acid had pH beyond the acceptable range indicating that the system breaks down at this concentration of shikimic acid. It should be noted that within 7 days pH of the gel sample comprising 0.1% shikimic acid also ran beyond the acceptable range; however, within 19 days pH returned into acceptable range and was within this range up to the end of experiment.

Regarding the gel viscosity the unexpected technical result was found by the Authors: shikimic acid at concentration range from 0.001% through 0,1% facilitated the gel thickening and served as the formulation stabilizer without changing the formulation pH within the normal range. Figure 1 demonstrates the change of gel viscosity versus gel concentration in accelerated ageing samples. One can see that addition of shikimic acid to 0.1% level is associated with increasing gel viscosity. At 0.5% the gel viscosity considerably drops, however, it is still within the normal viscosity range as described above.

The use of shikimic acid as stabilizer was not previously described in literature. On the contrary, it was reported that shikimic acid being a natural organic acid promotes the decline of the formulation stability [*Taofiq et al., 2017; Bartosz and Irene, 2019*]. The obtained technical results allow to use shikimic acid at wide concentration range as an ingredient of the laundry gel, in particular, as a stabilizer (Fig. 1).

Thus, the obtained results suggest that the laundry gel supplemented by shikimic acid at concentration range from 0.001% through 0,25% inclusively was stable for 32 days.

**Example 12.**

The laundry conditioner was used as the reference formulation for assessment of stability of conditioners supplemented by 0.001%, 0.025% shikimic acid.

The following stability parameters were used:

1. Dynamic viscosity - DV2T Brookfield viscosimeter was used for the measurements;
2. pH - measured by S220-Kit pH-meter/ion meter equipped with InLab Expert Pro-ISM electrode

The measurements were carried out at the baseline (point 0, control) and 14 days after conditioner preparation at three temperature regimens: K - measurements at room temperature t=20-25°C, T - measurements in thermostat at t=43°C, X - measurements at the cold chamber at t=2-8°C. The samples 1-6 comprise 0.001% shikimic acid, the samples 7-8 comprise 0.025% shikimic acid. The test results are summed up below.

Table 13. Test for dynamic viscosity of the laundry conditioner.

| No / T | 1_0 | 1_14 | 2_0 | 2_14 | 3_0 | 3_14 | 4_0 | 4_14 | 5_0 | 5_14 | 6_0 | 6_14 | 7_0 | 7_14 | 8_0 | 8_14 |
|---|---|---|---|---|---|---|---|---|---|---|---|---|---|---|---|---|
| К | | 56 | | 61 | | 53 | | 56 | | 33 | | 35 | | 56 | | 47 |
| Т | 52 | 56 | 56 | 62 | 52 | 54 | 55 | 59 | 37 | 38 | 35 | 40 | 63 | 56 | 46 | 46 |
| X | | 58 | | 63 | | 55 | | 56 | | 37 | | 39 | | 61 | | 50 |

N_0 и N_14, wherein N ∈ [1;8], are the measurements at baseline and at Day 14, respectively.

Units – Pa*s.

Table 14. Test for pH of laundry conditioner

| No / T | 1_0 | 1_14 | 2_0 | 2_14 | 3_0 | 3_14 | 4_0 | 4_14 | 5_0 | 5_14 | 6_0 | 6_14 | 7_0 | 7_14 | 8_0 | 8_14 |
|---|---|---|---|---|---|---|---|---|---|---|---|---|---|---|---|---|
| К | | 2.6 | | 2.6 | | 2.5 | | 2.8 | | 2.4 | | 2.5 | | 2.4 | | 2.7 |
| Т | 2.5 | 2.5 | 2.6 | 2.5 | 2.5 | 2.5 | 2.7 | 2.8 | 2.5 | 2.4 | 2.5 | 2.4 | 2.5 | 2.5 | 2.7 | 2.7 |
| X | | 2.5 | | 2.6 | | 2.5 | | 2.7 | | 2.4 | | 2.4 | | 2.4 | | 2.6 |

N_0 и N_14, wherein N ∈ [1;0], are the measurements at baseline and at Day 14, respectively.

For statistical processing of the data the software GraphPad Prism version 8.4.0 for macOS (GraphPad Software, USA) was used. Shapiro-Wilk test statistics was implemented to assess the normality of distribution. One-way analysis of variance was used to assess the statistical significance between the samples. The results obtained both for dynamic viscosity and for pH did not reveal statistically significant difference between the samples. These findings show that despite the literature data shikimic acid does not change the stability of the final product, and the conditioner comprising shikimic acid in the range from 0.001% to 0.025% at Day 14 has maintained viscosity and pH values at the baseline levels.

**Example 13.**

The laboratory test assessed the neutralization efficacy against unpleasant food odors in the process of washing with the use of liquid all-purpose laundry washing detergent comprising shikimic acid. Liquid all-purpose laundry washing detergent for white and color clothes shown in Table 1 was taken as a reference, and was supplemented by shikimic acid.

The test procedure used to assess neutralization of unpleasant odors by organoleptic method was based on scientific paper *Ikeura H. Deodorizing ability of Houttuynia cordata Thunb. (Dokudami) for masking garlic odor, 2012* . Intensive food odors in ethanolic solution were selected as the sources of unpleasant odors: garlic extract and sea herring extract, and also butyric acid entering the compositions of fatty acids in sweat and sebum, 10% ammonium solution, and instant coffee solution. This experiment allows to assess the neutralization efficacy against unpleasant food odors on cotton and synthetic fabrics under conditions simulating the washing process.

The test was carried out on the pieces of cotton tissue sized 4.5cm x 9.0cm pretreated with extracts, which were applied as the spots by a single spraying (garlic extract, sea herring extract, butyric acid) or 5-fold spraying (instant coffee) at the distance of 10-15 cm from the tissue piece. Ammonium solution was applied in the amount of one drop directly on tissue. Then the tissue pieces were air-dried horizontally for 5 minutes. One tissue piece was not treated and was used as control. Then the samples were washed in washing machine for 20 minutes. After washing the samples were air-dried horizontally until completely dry at room temperature.

Organoleptic method was used to compare the odors; baseline tissue odor (control) was compared with the odors of tissue samples after washing. The baseline odor intensities were rated as follows:

5 - no odor;
4 - faint odor;
3 - appreciable odor;
2 - intensive odor;
1 - very intensive odor.

The washing results were assessed by odor using 5-point scale compared with the baseline:

5 - no odor;
4 - faint odor;
3 - appreciable odor;
2 - the odor is a little less intensive in relation to the baseline;
1 - almost no difference in odors.

Results.

The assessment of odor neutralization efficacy showed marked deodorizing effect of shikimic acid added to liquid all-purpose laundry washing detergent for white and color clothes. Single washing lasted for 20 minutes resulted in elimination of unpleasant odors by 60-100% depending on shikimic acid concentration and the type of stain. A single washing of stained tissues using the reference formulation without shikimic acid did not provide such efficacy against the sea herring stain. The intensity of garlic odor decreased at higher shikimic acid concentrations. The greatest efficacy in respect to odor neutralization was achieved at the highest (0.5%) concentration of shikimic acid (Table 15).

Table 15. Assessment of odor neutralization efficacy.

| Test sample | Odor intensity, a.u. | | | | | | | | | | | |
| --- | --- | --- | --- | --- | --- | --- | --- | --- | --- | --- | --- | --- |
| | Control | | Ammonium hydroxide | | Herring | | Coffee | | Butyric acid | | Garlic | |
| | before | after | before | after | before | after | before | after | before | after | before | after |
| Sample No 1 Reference | 5 | 5 | 1 | 4 | 1 | 2 | 1 | 4 | 1 | 4 | 1 | 3 |
| Sample No 2 Reference + SA 0.001% | 5 | 5 | 1 | 5 | 1 | 3 | 1 | 5 | 1 | 4 | 1 | 3 |
| Sample No 3 Reference + SA 0.01% | 5 | 5 | 1 | 5 | 1 | 4 | 1 | 5 | 1 | 5 | 1 | 3 |
| Sample No 2 Reference + SA 0.1% | 5 | 5 | 1 | 5 | 1 | 4 | 1 | 5 | 1 | 5 | 1 | 4 |
| Sample No 3 Reference + SA 0.5% | 5 | 5 | 1 | 5 | 1 | 4 | 1 | 5 | 1 | 5 | 1 | 4 |

SA – shikimic acid.

The composition of all-purpose laundry washing detergent for white and color clothes supplemented by shikimic acid allows to effectively eliminate the odors from fabric surfaces thus improving organoleptic profile of the clothes.

Shikimic acid improves total deodorizing efficacy against common unpleasant food odors, such as fish oil (sea herring) and garlic odors.

**Claims**

1. The use of shikimic acid in the manufacture of a cleaning composition, said cleaning composition comprising 0.001-0.50% wt. of said shikimic acid and wherein the pH of said cleaning composition is in the range of 2.5-12.

2. The use of claim 1, wherein said cleaning composition is selected from the group consisting of

   - a laundry washing detergent or conditioner;
   - a dishwashing cleaner, including for use in manual and automatic dishwashing;
   - a surface cleaner, wherein said surface cleaner is for cleaning an outer dense or hard surface of a non-living object;
   - a hair and body cleaner, including for a living or non-living subject's hair and body cleaning; and
   - an acne or pimple cleaner, including for a mammal, wherein said mammal is preferably a human.

3. A cleaning composition, said cleaning composition comprising 0.001-0.50% wt. shikimic acid wherein the pH of said cleaning composition is in the range of 2.5-12.

4. The cleaning composition of claim 3, wherein said cleaning composition is selected from the group consisting of

   - a laundry washing detergent or conditioner;
   - a dishwashing cleaner, including for use in manual and automatic dishwashing; and
   - a surface cleaner, wherein said surface cleaner is for cleaning an outer dense or hard surface of a non-living object;
   - a hair and body cleaner, including for a living or non-living subject's hair and body cleaning; and
   - an acne or pimple cleaner, including for a mammal, wherein said mammal is preferably a human.

5. The use or cleaning composition of any of the preceding claims, said cleaning compositing not comprising acids selected from the group consisting of lactic acid, glycolic acid, lactobionic acid, ferulic acid, succinic acid, malic acid, azelaic acid, and gluconolactone.

6. The use or cleaning composition of any of the preceding claims, said cleaning composition further comprising at least one enzyme selected from the group consisting of an amylase, a lipase and a protease, preferably an amylase and/or a lipase.

7. The use or cleaning composition of any of the preceding claims, said cleaning composition comprising shikimic acid in an amount of 0.0010% wt., 0.0025% wt., 0.0050% wt., 0.010% wt., 0.015% wt., 0.020 % wt., 0.025% wt., 0.030% wt., 0.035% wt., 0.040% wt., 0.045% wt., 0.050% wt., 0.10% wt., 0.25% wt., 0.30% wt., 0.40% wt., 0.50% wt., or any values in between; or any ranges based on any combination of said values, including 0.001-0.025% wt., 0.001-0.100% wt., and 0.10-0.50% wt.

8. The use or cleaning composition of any of the preceding claims, wherein said cleaning composition is

   i) a formulation selected from the following: powder, granulate, capsule, tablet, foam, spray; or
   ii) a formulation selected from the following: liquid; solution, including aqueous glycerol, aqueous sorbitol, aqueous glycerol-sorbitol or aqueous propylene-glycol solutions; foam; spray; paste; gel; suspension; wherein

      - if said formulation is a hair and body cleaner as defined in claims 2 and 4, said hair and body cleaner is a shampoo, conditioner or balsam;
      - if said formulation is an acne or pimple cleaner as defined in claims 2 and 4, said cleaner is comprised by a patch.

9. The use or cleaning composition of any of the preceding claims, said shikimic acid having the CAS 138-59-0 registration number.

10. The use or cleaning composition of any of the preceding claims, wherein said cleaning composition is an aqueous manual hand dishwashing solution for dishwashing

   - at water hardness less than 42 dH, preferably 0-15dH,
   - at any temperature from +10°C to +45°C, and
   - preferably at pH=6.0-9.0, further preferably at pH=6.5-8.5.

11. The use or cleaning composition of any of claims 1-9, wherein said cleaning composition is an aqueous automatic dishwashing solution, for dishwashing in an automatic dishwasher

   - at water hardness less than 42 dH, preferably 0-15dH,
   - at any temperature from +40°C to +75°C, +40°C to +65°C, +40°C to +60°C, +40°C to +45°C, or +35°C to +45°C, and
   - preferably at pH=6.0-12.0, further preferably at pH=6.5-8.5.

12. The use or cleaning composition of any of claims 1-9, said surface cleaner for cleaning said surfaces, including metallic, polymeric, enameled, glass and wooden surfaces,

   - at any temperature from +10°C to +35°C, and
   - preferably at pH=6.0-9.0, further preferably at pH= 6.5-8.5.

13. The use or cleaning composition of any of claims 1-9, said laundry washing detergent or conditioner for hand and/or machine laundry washing and/or conditioning

   - at water hardness less than 42 dH, preferably 0-15dH,
   - at any temperature from +15°C to +75°C, including 15°C to +60°C, +40°C to +75°C, +40°C to +65°C, +40°C to +60°C, +40°C to +45°C, or +35°C to +45°C, and
   - preferably at pH=6.0-9.0, further preferably at pH=6.5-8.5.

14. The use or cleaning composition of claim 13, said laundry comprising clothes, fabrics, and textiles, wherein said clothes, fabrics and textiles are preferably selected from the group consisting of: cotton, synthetic, membrane, wool, silk, cashmere, merino, down, feather and mixtures thereof.

15. The use or cleaning composition of claim 13 or claim 14, said laundry comprising stain selected from the group consisting of mineral oil, soot, sebum, skin stains, blood, milk, ink, grass, chocolate drink, tea, red wine, coffee, and blackcurrant juice.

Laundry gel viscosity versus shikimic acid concentration after storage at +40°C for 25 days.
SA – shikimic acid.

**Fig. 1**

## EUROPEAN SEARCH REPORT

Application Number

EP 24 16 1084

### DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | IT MI20 082 180 A1 (SINERGA S P A) 11 June 2010 (2010-06-11) * page 12, lines 5-12; examples 1-4 * | 1-5,7-9 | INV. C11D3/20 A61K8/365 A61P17/10 A61Q5/00 |
| X | IT 2018 0002 0812 A1 (SINERGA GROUP S R L [IT]) 21 June 2020 (2020-06-21) * section 3.3, paragraph 2; examples mouthwash and tooth-paste * | 1,5,7-9 | A61Q5/12 A61Q19/10 C11D7/26 D06M13/207 |
| X | KR 2011 0028745 A (BIO FD & C [KR]) 22 March 2011 (2011-03-22) * claims 1-3; table 1 * | 1-5,7-9 | |
| X | US 11 911 489 B2 (SANJEEVITA BY AUDUBON LLC [US]) 27 February 2024 (2024-02-27) * column 8, lines 59,60; column 4, lines 39-55; examples 3-6 * | 1-5,7-9 | |
| X | CN 106 010 808 A (SHANDONG SUNWAY LANDSCAPE TECH CO LTD) 12 October 2016 (2016-10-12) * section background technique; claim 2; example 1 * | 1-9,12 | TECHNICAL FIELDS SEARCHED (IPC) C11D D06Q A61K A61P |
| X | Slavica: "SLAVICA - ECO Anise kitchen soap", , 1 January 2018 (2018-01-01), pages 1-6, XP093184169, Retrieved from the Internet: URL:https://slavica.eu/en/solvgreen-produk ty-en/308-eco-anise-kitchen-soap [retrieved on 2024-07-10] * pages 1 and 2 * | 1-4, 7-11, 13-15 | A61Q D06M |

The present search report has been drawn up for all claims

| Place of search | Date of completion of the search | Examiner |
|---|---|---|
| The Hague | 22 July 2024 | Douelle, Frédéric |

EPO FORM 1503 03.82 (P04C01)

## ANNEX TO THE EUROPEAN SEARCH REPORT
## ON EUROPEAN PATENT APPLICATION NO.

EP 24 16 1084

22-07-2024

| Patent document cited in search report | Publication date | Patent family member(s) | | Publication date |
|---|---|---|---|---|
| IT MI20082180 A1 | 11-06-2010 | NONE | | |
| IT 201800020812 A1 | 21-06-2020 | ----------- | | ---------- |
| KR 20110028745 A | 22-03-2011 | NONE | | |
| US 11911489 B2 | 27-02-2024 | US | 2019076338 A1 | 14-03-2019 |
| | | WO | 2017197396 A1 | 16-11-2017 |
| CN 106010808 A | 12-10-2016 | NONE | | |

EPO FORM P0459

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Non-patent literature cited in the description**

- **GHOSH S. et al.** Production of shikimic acid. *Biotechnology advances*, 2012
- **BATORY M,** ; **ROTSZTEJN H.** Shikimic acid in the light of current knowledge. *J Cosmet Dermatol.*, February 2022, vol. 21 (2), 501-505
- **MUNK S et al.** *Primary odorants of laundry soiled with sweat/sebum: Influence of lipase on the odor profile*, 2000
- **BATORY M.,** ; **ROTSZTEJN H**. *Shikimic acid in the light of current knowledge*, 2022
- **BAI J. et al.** *Antibacterial activity of shikimic acid from pine needles of Cedrus deodara against Staphylococcus aureus through damage to cell membrane*, 2015
- **BAI J et al.** *Comparative study on antibacterial mechanism of shikimic acid and quinic acid against Staphylococcus aureus through transcriptomic and metabolomic approaches,*, 2022

- **BORAH J. C.** *Shikimic acid: a highly prospective molecule in pharmaceutical industry*, 2015
- **TAOFIQ O et al.** Hydroxycinnamic acids and their derivatives:. *Cosmeceutical significance, challenges and future perspectives, a review*, 2017
- *CHEMICAL ABSTRACTS*, 138-59-0
- **LI X. et al.** *A review of the role of sebum in the mechanism of acne pathogenesis*, 2017
- **CAO J. et al.** *Elucidation of alpha-amylase inhibition by natural shikimic acid derivates regarding the infrequent uncompetitive inhibition mode and structure-activity relationship,*, 2023
- **RAW AT G. et al.** *An interactive study of influential parameters for shikimic acid production using statistical approach, scale up and its inhibitory action on different lipases*, 2013